# EUROPÄISCHE PATENTSCHRIFT

(11) **EP 3 085 401 B1**
(45) Veröffentlichungstag und Bekanntmachung des Hinweises auf die Patenterteilung: **24.07.2019**
(21) Anmeldenummer: 15164985.2
(22) Anmeldetag: 24.04.2015
(51) Int. Cl.: A61M 1/30, A61M 1/34

(54) **HÄMODIALYSEGERÄT**
HAEMODIALYSIS DEVICE
APPAREIL D'HÉMODIALYSE

(43) Veröffentlichungstag der Anmeldung: 26.10.2016
(73) Patentinhaber: D.Med Consulting GmbH, 20359 Hamburg (DE)
(72) Erfinder: Breuch, Gerd, 53844 Troisdorf (DE); Biermann, Frank, 22455 Hamburg (DE); Breuch, Julius, 20457 Hamburg (DE); Holzschuh, Robert, 22301 Hamburg (DE)
(74) Vertreter: Patentanwälte ter Smitten Eberlein-Van Hoof Rütten Partnerschaftsgesellschaft mbB

(56) Entgegenhaltungen:
- EP-A1- 2 140 889
- EP-A1- 2 666 491
- US-A- 3 902 490
- US-A- 5 011 607
- US-A- 5 536 412

## Beschreibung

Die Erfindung bezieht sich auf ein Hämodialysegerät für die Durchführung einer sog. Single-Needle Hämodialyse-Behandlung.

Ein Hämodialysegerät nach dem Stand der Technik weist einen Dialysatkreis, einen Blutkreis und einen Dialysator zwischen dem Dialysatkreis und dem Blutkreis auf. Der Dialysator weist eine Dialysatkammer, die fluidisch in den Dialysatkreis eingebunden ist, und eine Blutkammer auf, die fluidisch in den Blutkreislauf eingebunden ist. Zum Dialysatkreis gehört ferner eine Dialysatquelle, von der eine Dialyseflüssigkeit zur Verfügung gestellt wird, eine Förderpumpe zwischen der Dialysatquelle und der Dialysatkammer zum Fördern der Dialyseflüssigkeit zum Dialysator und eine Abfallpumpe stromabwärts der Dialysatkammer des Dialysators, Der Blutkreis eines Hämodialysegeräts weist eine Saugleitung auf, die zu einem Einlass der Dialysator-Blutkammer führt und weist eine Rücklaufleitung auf, die von einem Auslass der Dialysator-Blutkammer wegführt. In dem Blutkreis ist im Verlauf der Saugleitung eine Blutpumpe angeordnet. Ferner ist im Verlauf der Rücklaufleitung ein Leitungsventil angeordnet, durch das die betreffende Rücklaufleitung fluidisch gesperrt oder geöffnet werden kann. Ein derartiges Hämodialysegerät ist aus EP 2666491 A1 bekannt.

Bei der herkömmlichen Dialysebehandlung wird die Saugleitung über eine Nadel fluidisch mit einem Blutgefäß des Patienten verbunden und wird die Rücklaufleitung über eine zweite Nadel mit einem Blutgefäß des Patienten fluidisch verbunden. Auf diese Weise kann das von dem Patienten durch die Saugleitung angesaugte Patientenblut kontinuierlich in dem Blutkreis gefördert werden. Allerdings müssen hierzu stets zwei Nadeln an dem Patienten appliziert werden, was sich quantitativ unmittelbar auf die Schmerzen des Patienten während der Nadel-Punktion, die Infektionsgefahr und den Verschleiß eines gegebenenfalls vorhandenen Patientenshunts auswirkt.

Alternativ zu der Zwei-Nadel Dialysebehandlung kann die sogenannte Single-Needle Dialysebehandlung durchgeführt werden, bei der nur eine einzige Nadel an einem Patienten-Blutgefäß appliziert wird. Die Single-Needle Dialysebehandlung wird auch und insbesondere dann angewendet, wenn sich während einer Zwei-Nadel Dialysebehandlung einer der beiden Zugänge verschließt, beispielsweise verstopft.

Bei der Single-Needle Dialysebehandlung werden die Saugleitung und die Rücklaufleitung beide patientennah an eine Leitungsverzweigung, ein sogenanntes Y-Stück, angesteckt, wobei sich an die Leitungsverzweigung eine einzige Gefäßanschluss-Leitung bzw. -Nadel anschließt. Die Gefäßanschluss-Leitung bzw. -Nadel dient also sowohl zum Ansaugen des Bluts von dem patientenseitigen Blutgefäß als auch zum Rückführen des Bluts in das Blutgefäß. Ein Hämodialysegerät für die Sinlge-Needle Hämodialysebehandlung ist aus US 5536412 A bekannt.

Da bei einer Single-Needle Dialysebehandlung nur noch ein einziger Blutgefäß-Zugang zur Verfügung steht, muss das Patientenblut über diesen einen Blutgefäß-Zugang abwechselnd angesaugt und zurückgepumpt werden. Dies erfolgt bei konventionellen Single-Needle Hämodialysegeräten dadurch, dass in der Ansaugphase bei geschlossenem Leitungsventil, das stromabwärts der Blutpumpe angeordnet ist, zunächst Patientenblut in die Leitungen gepumpt wird, die sich hierbei aufgrund ihrer Elastizität über ihr Nominalvolumen hinaus ausdehnen. Die Ansaugphase wird druckgesteuert oder zeitgesteuert beendet, und das Leitungsventil geöffnet, wodurch die Rücklaufphase eingeleitet wird. In der Rücklaufphase wird das in den Leitungen unter Druck gespeicherte Blutvolumen durch die sich elastisch wieder auf ihr Nominalvolumen zusammenziehenden Leitungen durch die Gefäßanschluss-Leitung in das Patienten-Blutgefäß ausgeschoben, also zurückgepumpt. Das Umschalten auf die Ansaugphase erfolgt ebenfalls druckgesteuert oder zeitgesteuert. Dieses Single-Needle Dialyseverfahren ist sehr einfach und kann insbesondere während einer Zwei-Nadel Dialysebehandlung unter Zuhilfenahme und Einbau der Leitungsverzweigung schnell und einfach eingerichtet werden, wobei die übrigen blutseitigen Leitungen weiter verwendet werden können.

Das Pumpvolumen, bezogen auf eine Ansaugphase, bestimmt sich im Wesentlichen aus der Elastizität und dem absoluten Nominalvolumen der blutseitigen Leitungen, und liegt bei herkömmlichen Zwei-Nadel Hämodialyse-Leitungen in der Größenordnung von 100 ml oder deutlich darunter. Wegen des relativ geringen Pumpvolumens während einer Ansaugphase ist die therapeutische Effektivität der Single-Needle Dialysebehandlung, also die zeitbezogene Clearanceleistung, vergleichsweise gering.

Es sind aus dem Stand der Technik zwar eine Reihe von Maßnahmen bekannt, durch die das Ansaugvolumen vergrößert werden kann, jedoch sind diese Maßnahmen in der Regel aufwendig und erfordern häufig spezielle Ansaug- und Rücklaufleitungen, was wiederum eine Umrüstung von einem Zwei-Nadel Dialysebetrieb auf einen Single-Needle Dialysebetrieb während einer Dialysebehandlung kompliziert gemacht und erhebliche Zusatzkosten verursacht.

Vor diesem Hintergrund ist es Aufgabe der Erfindung, ein Hämodialysegerät zu schaffen, bei dem mit einfachen Mitteln die sogenannte Clearanceleistung, also die Blut-Reinigungsleistung, verbessert ist.

Diese Aufgabe wird erfindungsgemäß gelöst mit einem Hämodialysegerät mit den Merkmalen des Anspruchs 1.

Das erfindungsgemäße Hämodialysegerät weist im Dialysatkreis eine Dialysatquelle auf, von der eine Dialyseflüssigkeit zur Verfügung gestellt wird. Die Dialysatquelle kann mehrere Komponenten aufweisen, beispielsweise einen Dialysewasser-Tank und einen Behälter mit DialysatKonzentrat. Zwischen der Dialysatquelle und dem Dialysator ist eine Förderpumpe zum Fördern der Dialyseflüssigkeit von der Dialysatquelle zu einer Dialysatkammer des Dialysators angeordnet. Stromabwärts des Dialysators ist eine Abfallpumpe angeordnet, die durch eine Ablaufleitung fluidisch mit der Dialysatkammer des Dialysators verbunden ist. Die Förderpumpe und die Abfallpumpe sind bevorzugt mechanisch oder elektronisch miteinander zu einer Bilanzierpumpe gekoppelt, so dass die Fördermenge der Förderpumpe stets der Fördermenge der Abfallpumpe entspricht. Zwischen der Förderpumpe und der Abfallpumpe wird auf diese Weise ein konstantes Dialysatvolumen eingeschlossen.

Im Dialysatkreis ist fluidisch zwischen der Förderpumpe und der Abfallpumpe ein von der Ablaufleitung abzweigendes separates Oszillationsvolumen angeordnet. Das Oszillationsvolumen kann mechanisch passiv ausgebildet sein, und wird beispielsweise durch entsprechende externe Druckverhältnisse gefüllt und geleert. Es kann auch ein über ein entsprechend geschaltetes Ventil zugängliches passives Volumen sein, das beispielsweise elastisch ausgebildet ist, um das Füllen oder Leeren zu vereinfachen.

Besonders bevorzugt ist das Oszillationsvolumen stromabwärts der Dialysatkammer angeordnet, ist also zwischen der Dialysatkammer und der Abfallpumpe angeordnet.

Das Oszillationsvolumen wird bevorzugt von einer Oszillationspumpe gebildet, worunter vorliegend allgemein jede Pumpe zu verstehen ist, die in beide Richtungen pumpen kann und die ausreichend schnell zwischen den beiden Pumprichtungen umgeschaltet werden kann. Die Oszillationspumpe kann beispielsweise eine sogenannte Ultrafiltrationspumpe sein, die fluidisch parallel zu der Abfallpumpe angeordnet ist. Die Oszillationspumpe ist besonders bevorzugt jedoch als Hubpumpe, beispielsweise als Kolbenpumpe oder als Membranpumpe ausgebildet, die das Fluidvolumen in dem Dialysatkreis zwischen der Förderpumpe und der Abfallpumpe kurzfristig erhöhen und das auf diese Weise zwischengespeicherte Flüssigkeitsvolumen wieder in den Dialysatkreis zurückgeben kann. Das Oszillationsvolumen bzw. die Oszillationspumpe ist dazu bevorzugt Teil einer toten Abzweigung, also nicht Teil einer Förderschleife parallel zu der Abfallpumpe. Durch das Oszillationsvolumen bzw. die Oszillationspumpe wird sichergestellt, dass das zugeführte Flüssigkeitsvolumen exakt dem wieder abgeführten Flüssigkeitsvolumen entspricht.

In dem Blutkreis ist das Leitungsventil im Verlauf der Rücklaufleitung angeordnet.

Erfindungsgemäß ist ferner eine Gerätesteuerung vorgesehen, die das Füllen und Leeren des Oszillationsvolumens und das Schalten des Leitungsventils miteinander abstimmt. Besonders bevorzugt weist die Gerätesteuerung einen Synchronisierer auf, der das Leitungsventil und das Oszillationsvolumen bzw. die Oszillationspumpe mit demselben Arbeitstakt ansteuert, so dass die Schließfrequenz des Leitungsventils und die Oszillationsfrequenz der Oszillationspumpe annähernd gleich sind. Die Oszillationspumpe und das Leitungsventil werden jedoch nicht notwendigerweise jeweils zum selben Zeitpunkt, also gleichzeitig bzw. gleichphasig, umgeschaltet.

Während der Ansaugphase schließt die Gerätesteuerung bzw. der Synchronisierer das Leitungsventil, das im Verlauf der Rücklaufleitung fluidisch stromabwärts der Dialysator- Blutkammer angeordnet ist, und betreibt die Oszillationspumpe im Saugmodus. Gleichzeitig läuft die Blutpumpe und pumpt Blut aus dem Patienten-Blutgefäß über die Nadel, die Gefäßanschlussleitung, und die Leitungsverzweigung in die Ansaugleitung, die Blutkammer des Dialysators und in die Rücklaufleitung bis zu dem geschlossenen Leitungsventil. Die Blutpumpe füllt den Blutkreis mit Blut, der sich dabei im Bereich der Ansaugleitung und der Rücklaufleitung elastisch ausdehnt. Das Volumen, das durch die elastische Ausdehnung der Leitungen beim Ansaugen des Blutes entsteht, ist das Leitungs-Pumpvolumen. Gleichzeitig hierzu saugt die sich im Saugmodus befindende Oszillationspumpe Flüssigkeit aus dem Dialysatkreis ab, was dazu führt, dass eine entsprechende Menge an Flüssigkeit aus dem Patientenblut in der Blutkammer des Dialysators über die Dialysatormembran in die Dialysatkammer ultrafiltriert wird. Die Ansaug-Flussrate sollte hierbei zu jedem Zeitpunkt nicht größer sein als die Flussrate der aus der Dialysatkammer ausfließenden Flüssigkeit, so dass sichergestellt ist, dass kein Rückfluss von verbrauchtem Dialysat zur Blutseite hin auftreten kann.

Sobald die Ansaugphase beendet ist, was beispielsweise zeitgesteuert oder druckgesteuert ausgelöst wird, startet die Rücklaufphase. In der Rücklaufphase öffnet der Synchronisierer das Rücklauf-Leitungsventil, so dass das in den elastischen Leitungen unter Druck stehende Blut zu der Leitungsverzweigung und in die Gefäßanschluss-Leitung strömt, von wo aus das Blut durch die Nadel in das Patienten-Blutgefäß strömt. Gleichzeitig wird die Oszillationspumpe durch den Synchronisierer vom Saugmodus in den Rückgabemodus umgeschaltet, so dass Dialyseflüssigkeit aus der Dialysatkammer durch die Dialysatormembran in die Blutkammer gedrückt wird. Da die Förderpumpe und die Abfallpumpe während der Rücklaufphase weiterpumpen, ist sichergestellt, dass nicht das soeben in der Saugphase aus dem Patientenblut durch die Dialysatormembran abgesaugte Ultrafiltrat wieder in den Blutkreis zurückgepumpt wird, sondern ausschließlich frische Dialyseflüssigkeit in den Blutkreislauf zurückgepumpt wird, das ständig in die Dialysatkammer nachfließt. Das während der Saugphase aus dem Blutkreis abgesaugte Flüssigkeitsvolumen wird auf diese Weise also durch Dialyseflüssigkeit substituiert.

Mit einer einfachen Maßnahme, nämlich einer einfachen Oszillationspumpe auf der Dialysatseite und einem Synchronisierer, der die Schließfrequenz des Leitungsventils und die Oszillationsfrequenz der Oszillationspumpe miteinander synchronisiert, kann ohne Wechsel der blutkreisseitigen Leitungen von einem Zwei-Nadel Dialysebetrieb auf einen Single-Needle Dialysebetrieb gewechselt werden. Für den Zwei-Nadel Dialysebetrieb und den Single-Needle Dialysebetrieb kann also dasselbe Leitungs-Set verwendet werden, was in der Praxis bei einem Wechsel der Betriebsart während einer Dialysebehandlung eine erhebliche Vereinfachung darstellt. Ferner werden dadurch, dass für beide Betriebsarten praktisch dasselbe blutseitige Leitungs-Set verwendet werden kann, die Beschaffungs- und die Lagerungskosten erheblich reduziert.

Durch die Oszillationspumpe wird das gesamte Pumpvolumen während eines Pumpzyklus' erheblich vergrößert, so dass die prinzipbedingt relativ schlechte zeitbezogene Clearanceleistung beim Single-Needle Betrieb verbessert wird.

Vorzugsweise arbeitet die Oszillationspumpe mit einem konstanten Hubvolumen, das zwischen 0,5 ml und 60 ml liegt, und besonders bevorzugt zwischen 3 ml und 30 ml beträgt. Das Hubvolumen der Oszillationspumpe ist bevorzugt konstant und kann nicht variiert werden. Hierdurch ist es möglich, eine relativ einfach konstruierte, zuverlässige und preiswert beschaffbare Pumpe als Oszillationspumpe einzusetzen, beispielsweise eine einfache Membranpumpe.

Vorzugsweise ist die Blutpumpe als eine peristaltische Schlauchpumpe ausgebildet, wie sie im Blutkreis üblich ist. Die peristaltische Schlauchpumpe erlaubt eine einfache Applikation der Ansaugleitung, verhindert zuverlässig Rückfluss und erlaubt eine ausreichend genaue Dosierung der Flussrate.

Vorzugsweise sind zwei Leitungsventile in dem Blutkreis vorgesehen, nämlich das erste Leitungsventil im Verlauf der Rücklaufleitung und ein zweites Leitungsventil im Verlauf der Saugleitung stromaufwärts der Blutpumpe und stromabwärts der Blutkammer des Dialysators.

Das bzw. die Leitungsventile sind besonders bevorzugt als elektrische Klemmventile ausgebildet, Durch das Vorsehen eines zweiten Leitungsventils im Verlauf der Saugleitung wird der Rückfluss entgegen der Pumprichtung minimiert. Die beiden Leitungsventile werden gegensinnig zueinander geöffnet und geschlossen, d.h. bei geöffnetem Ansaug-Leitungsventil ist das Rücklauf -Leitungsventil geschlossen, und umgekehrt.

Vorzugsweise ist zwischen der Förderpumpe und der Dialysatkammer des Dialysators ein Sterifilter angeordnet. Durch den Sterilfilter wird sichergestellt, dass die von der Dialysatquelle kommende Dialysatflüssigkeit steril ist, wenn sie in die Dialysatkammer einströmt.

Beim Betreiben einer Single-Needle Hämodialyse-Behandlung mit dem vorbeschriebenen Hämodialysegerät wird die Blutpumpe mindestens während jeder Ansaugphase aktiviert. Während der Dauer der Dialysebehandlung schaltet der Synchronisierer das Rücklauf-Schaltventil mit einer Schließfrequenz und steuert die Oszillationspumpe mit einer Oszillationsfrequenz, die gleich der Schließfrequenz ist, jedoch nicht notwendigerweise phasengleich abläuft.

Das Rücklauf-Leitungsventil wird derart geschaltet, dass das Schließintervall des Rücklauf-Leitungsventils mindestens während seines halben Schließintervalls zusammenfällt mit dem Saugintervall der Oszillationspumpe, besonders bevorzugt mindestens während 75 % des Schließintervalls.

Ein Ansaug-Leitungsventil ist an der Saugleitung stromaufwärts der Blutpumpe angeordnet. Ferner ist vorgesehen, dass der Synchronisierer das Ansaug-Leitungsventil derart schaltet, dass das Schließintervall des Ansaug-Leitungsventils mindestens zu 70 % zusammenfällt mit dem Rückgabe-Intervall der Oszillationspumpe.

Im Folgenden wird ein Ausführungsbeispiel der Erfindung anhand der Zeichnungen näher erläutert. Es zeigen:
Figur 1 ein Hämodialysegerät für eine Single-Needle Dialysebehandlung während der Ansaugphase, und
Figur 2 das Hämodialysegerät der Figur 1 während der Rücklaufphase.

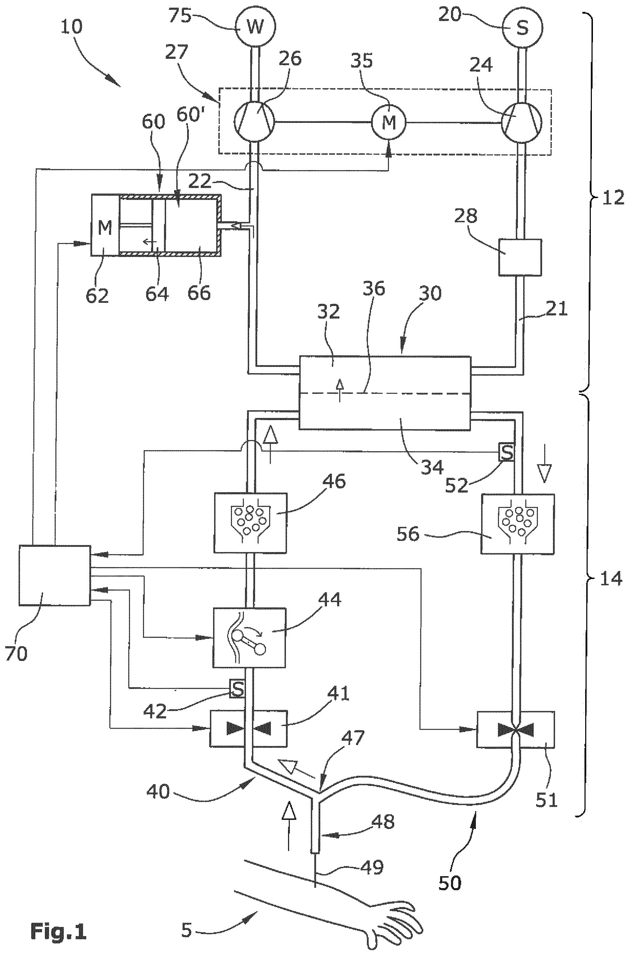

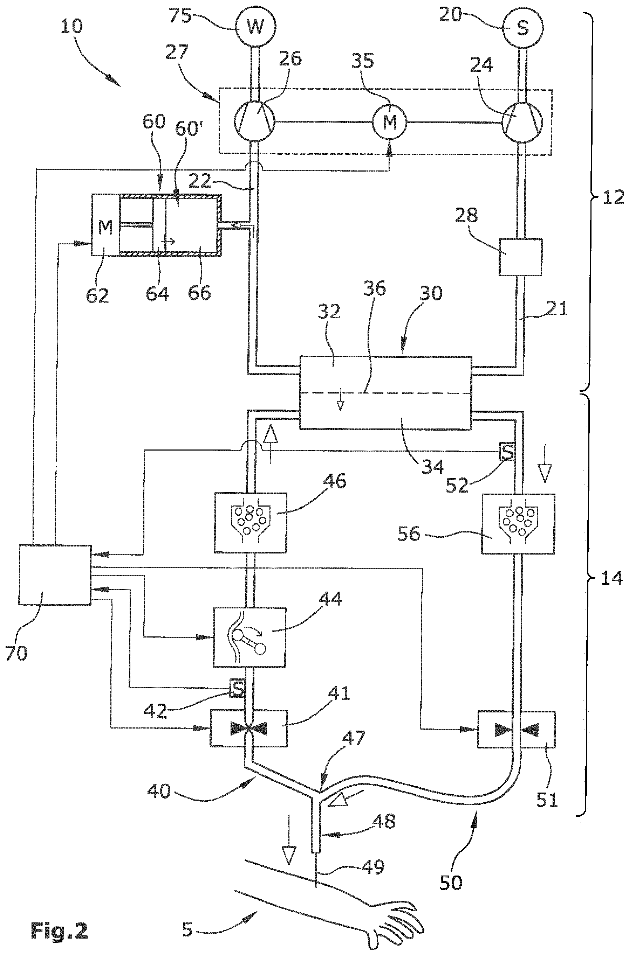

In den Figuren 1 und 2 ist ein Hämodialysegerät 10 für die Durchführung einer Single-Needle Hämodialyse-Behandlung dargestellt. Das Hämodialysegerät 10 weist einen Dialysatkreis 12, einen Blutkreis 14 und einen Dialysator 30 zwischen dem Dialysatkreis 12 und dem Blutkreis 14 auf. Alle Teile des Blutkreises 14 einschließlich des Dialysators 30 sind Wegwerf-Artikel, die nur einmal gebraucht werden.

Der Dialysatkreis 12 weist eine Dialysatquelle 20 auf, die die Dialyseflüssigkeit für die Durchführung der Dialyse liefert. Die Dialysatquelle 20 kann sich beispielsweise zusammensetzen aus einem Dialysewasser-Behälter und einem Dialysatkonzentrat-Behälter. Stromabwärts der Dialysatquelle 20 ist eine Förderpumpe 24 angeordnet, die die Dialyseflüssigkeit von der Dialysatquelle 20 über eine Zulaufleitung 21 zu dem Dialysator 30 pumpt. Im Verlauf der Zulaufleitung 21 stromabwärts der Förderpumpe 24 ist ein Sterifilter 28 angeordnet, durch den die Sterilität der zum Dialysator 30 fließenden Dialyseflüssigkeit sichergestellt wird. Es ist noch ein zweiter Sterifilter (nicht dargestellt) vorzusehen, um die Sterilität der in den Patienten infusionierten Dialysatflüssigkeit sicherzustellen. Der zweite Sterlfilter kann auch integraler Bestandteil des Dialysators 30 sein.

Der Dialysator 30 weist eine Dialysatkammer 32 auf der Dialysatseite und eine Blutkammer 34 auf der Blutseite auf. Die Dialysatkammer 32 ist durch eine Dialysatormembran 36 von der Blutkammer 34 getrennt. Die Dialysatkammer 32 führt eine Ablaufleitung 22 zu einer Abfallpumpe 26, die die Flüssigkeit in einen Abfallbehälter 75 pumpt. Die Förderpumpe 24 und die Abfallpumpe 26 sind mechanisch miteinander gekoppelt und bilden gemeinsam eine Bilanzierpumpe 27, durch die sichergestellt ist, dass die Förderrate der Förderpumpe 24 einerseits und der Abfallpumpe 26 andererseits stets gleich ist. Die beiden Pumpen 24,26 werden gemeinsam durch einen Pumpen-Antriebsmotor 35 angetrieben.

Von der Ablaufleitung 22 zweigt eine als Kolbenpumpe ausgebildete Oszillationspumpe 60 ab, die einen elektrischen Pumpenantrieb 62 und einen Pumpenkolben 64 aufweist, der sich in einem Hubvolumen 66 oszillierend bewegt. Das Hubvolumen 66 der Kolbenpumpe ist konstant, kann also nicht variiert werden. Das Hubvolumen 66 beträgt vorliegend beispielsweise ungefähr 10 ml. Es kann jedoch auch eine Oszillationspumpe mit verstellbarem Hubvolumen verwendet werden. Die Oszillationspumpe 60 bildet vorliegend ein Oszillationsvolumen 60'.

Zu dem Blutkreis 14 gehört eine Ansaugleitung 40, die von dem einen Arm einer Leitungsverzweigung 47 zu dem Einlass der Blutkammer 34 führt und gehört eine Rücklaufleitung 50, die von dem Auslass der Blutkammer 34 zu dem zweiten Arm der Leitungsverzweigung 47 führt. An den dritten Arm der Leitungsverzweigung 47 schließt sich eine Gefäßanschlussleitung 48 mit einer Nadel 49 an, die in ein Blutgefäß eines Patienten 5 appliziert ist. Die Nadel 49 kann selbstverständlich auch an einem künstlichen patientenseitigen Dialyseshunt appliziert sein.

Stromaufwärts der Leitungsverzweigung 47 sind im fluidischen Verlauf der Ansaugleitung 40 ein elektromagnetisches Leitungsventil 41, ein Drucksensor 42, eine peristaltische Blutpumpe 44 und eine Luftfalle 46 angeordnet. Das Ansaug-Leitungsventil 41 ist als elektromagnetisches Klemmventil ausgebildet. Die Blutpumpe 44 ist eine peristaltische Blutpumpe, in die die Ansaugleitung 40 eingelegt ist. Im Verlauf der Rücklaufleitung 50 ist stromabwärts der Blutkammer 34 ein zweiter Drucksensor 52, eine Luftfalle 56 sowie ein weiteres Leitungsventil 51 angeordnet, das ebenfalls als elektromagnetisches Klemmventil ausgebildet ist.

Für die Steuerung und Synchronisierung einiger Elemente des Hämodialysegeräts 10 ist als Teil einer Gerätesteuerung ein Synchronisierer 70 vorgesehen, der Teil einer Gerätesteuerung sein kann. Der Synchronisierer 70 kann in Form von Hardware realisiert sein, kann jedoch alternativ im Wesentlichen aus einem Computerprogramm bestehen. Der Synchronisierer 70 empfängt die Sensorsignale der beiden Drucksensoren 42,52 und steuert insbesondere die beiden Leitungsventile 41,51, die Blutpumpe 44, den Pumpen-Antriebsmotor 25 und die Oszillationspumpe 60.

In der Figur 1 ist das Hämodialysegerät 10 in der Ansaugphase dargestellt. In der Ansaugphase sind die Förderpumpe 24, die Abfallpumpe 26 und die Blutpumpe 44 aktiv. Ferner ist das Ansaug-Leitungsventil 41 geöffnet und ist das Rücklauf-Leitungsventil 51 geschlossen. Die Oszillationspumpe 60 ist in ihrer Saugphase, so dass Flüssigkeit aus der Ablaufleitung 22 in das Hubvolumen 66 der Oszillationspumpe 60 angesaugt wird.

In der Ansaugphase wird durch die Blutpumpe 44 das Blutkammer-Volumen und das Leitungsvolumen zwischen der Blutpumpe 44 und dem geschlossenen Rücklauf-Leitungsventil 51 durch das kompressible Luftvolumen in den Luftfallen 46, 56 und wegen der Elastizität der Leitungen 40,50 über ihr Nominalvolumen hinaus mit einem Überdruckvolumen an Blut gefüllt, und ein entsprechender Innen-Fluiddruck aufgebaut. Ferner wird in der Ansaugphase durch die Oszillationspumpe 60 Flüssigkeit aus dem Fluidvolumen zwischen der Förderpumpe 24 und der Abfallpumpe 26 abgesaugt, so dass Flüssigkeit aus der Blutkammer 34 in die Dialysatkammer 32 ultrafiltriert wird. Da die Förderpumpe 24 und die Abfallpumpe 26 hierbei kontinuierlich in Betrieb sind, wird das Ultrafiltrat in Richtung Abfallpumpe 26 aus der Dialysatkammer 32 heraus weggefördert. Das maximale Überdruckvolumen beträgt ca. 60 ml und das Hubvolumen ca. 10 ml, so dass das gesamte Pumpvolumen während eines Ansaug-/Rücklauf-Zyklus insgesamt ca. 70 ml beträgt.

Das Umschalten zwischen der Saugphase und der Rücklaufphase erfolgt vorliegend druckgesteuert. Die Ansaugphase wird beendet, sobald an dem Rücklauf-Drucksensor 52 eine eingestellte Rücklauf-Druckschwelle überschritten ist. Sobald dies durch den Synchronisierer 70 festgestellt wird, schaltet der Synchronisierer 70 in die Rücklaufphase, die in Figur 2 dargestellt ist, woraufhin das Ansaug-Leitungsventil 41 geschlossen und das Rücklauf-Leitungsventil 51 geöffnet wird. Die Blutpumpe 44 wird angehalten. Ferner wird die Oszillationspumpe 60 umgeschaltet in den Rückgabemodus, so dass die Flüssigkeit aus dem Hubvolumen 66 zurück in die Ablaufleitung 22 gepumpt wird. Hierdurch fließt die Hubvolumen-Menge an frischer Dialyseflüssigkeit, die durch die Förderpumpe 24 in die Dialysatkammer 32 gepumpt wird, durch die Dialysatormembran 36 in die Blutkammer 34, so dass die Volumenbilanz in dem Blutkreis 14 annähernd neutral bleibt. Das gesamte Pumpvolumen wird auf diese Weise durch die Rücklaufleitung 50, die Leitungsverzweigung 47, die Gefäßanschlussleitung 48 und die Nadel 49 zurück in das Patienten-Blutgefäß gepumpt.

Sobald der Rücklauf-Drucksensor 52 einen Fluiddruck detektiert, der unterhalb einer eingestellten konstanten Ansaug-Druckschwelle liegt, schaltet der Synchronisierer 70 von der Rücklaufphase wieder in die Ansaugphase.

Während der Ansaugphase wird der von dem Ansaug-Drucksensor 42 detektierte Fluiddruck durch den Synchronisierer 70 überwacht. Sobald ein unterer Fluiddruck unterschritten wird, reduziert der Synchronisierer 70 die Pumprate der Blutpumpe 44.

## Patentansprüche

1. Hämodialysegerät (10) für die Durchführung einer Single-Needle Hämodialyse-Behandlung, mit einem Dialysatkreis (12), einem Blutkreis (14) und einem Dialysator (30) zwischen dem Dialysatkreis (12) und den Blutkreis (14),
wobei der Dialysator (30) eine Dialysatkammer (32) und eine Blutkammer (34) aufweist,
wobei der Dialysatkreis (12) aufweist:
eine Dialysatquelle (20), von der eine Dialyseflüssigkeit zur Verfügung gestellt wird,
eine Förderpumpe (24) zwischen der Dialysatquelle (20) und dem Dialysator (30) zum Fördern der Dialyseflüssigkeit von der Dialysatquelle (20) zu einer Dialysatkammer (32) des Dialysators (30), und
eine Abfallpumpe (26) stromabwärts der Dialysatkammer (32) des Dialysators (30), wobei die Dialysatkammer (32) und die Abfallpumpe (26) durch eine Ablaufleitung (22) fluidisch miteinander verbunden sind,
wobei der Blutkreis (14) aufweist:
eine einzige Gefäßanschluss-Leitung (48), an die sich eine Leitungsverzweigung (47) anschließt, von der eine Saugleitung (40) zu einem Einlass der Dialysator-Blutkammer (34) und eine Rücklaufleitung (50) zu einem Auslass der Dialysator-Blutkammer (34) führt,
eine Blutpumpe (44) im Verlauf der Ansaugleitung (40), und
ein Leitungsventil (51) im Verlauf der Rücklaufleitung (50), wobei im Dialysatkreis (12) und fluidisch zwischen der Förderpumpe (24) und der Abfallpumpe (26) ein von der Ablaufleitung (22) abzweigendes separates Oszillationsvolumen (60') angeordnet ist,
und
wobei eine Gerätesteuerung vorgesehen ist, die das Schalten des Leitungsventils (51) und das Oszillieren des Oszillationsvolumens miteinander abstimmt.

2. Hämodialysegerät (10) nach Anspruch 1, wobei das Oszillationsvolumen (60') von einer angetriebenen Oszillationspumpe (60) gebildet wird.

3. Hämodialysegerät (10) nach Anspruch 1 oder 2, wobei die Gerätesteuerung einen Synchronisierer (70) aufweist, der das Leitungsventil (51) und die Oszillationspumpe (60) mit dem gleichen Arbeitstakt ansteuert, so dass die Schließfrequenz des Leitungsventils (51) und die Oszillationsfrequenz der Oszillationspumpe (60) gleich sind.

4. Hämodialysegerät (10) nach Anspruch 3, wobei die Oszillationspumpe (60) als Hubpumpe mit einem konstanten maximalen Hubvolumen ausgebildet ist.

5. Hämodialysegerät (10) nach Anspruch 4, wobei die Oszillationspumpe (60) als Kolbenpumpe oder als Membranpumpe ausgebildet ist.

6. Hämodialysegerät (10) nach einem der vorangegangenen Ansprüche, wobei die Oszillationspumpe (60) mit einem konstanten Hubvolumen arbeitet, das 0,5 ml bis 100 ml beträgt, besonders bevorzugt 3,0 ml -30 ml beträgt.

7. Hämodialysegerät (10) nach einem der vorangegangenen Ansprüche, wobei die Blutpumpe (44) als eine peristaltische Schlauchpumpe ausgebildet ist.

8. Hämodialysegerät (10) nach einem der vorangegangenen Ansprüche, wobei ein zweites Leitungsventil (41) an der Ansaugleitung (40) stromaufwärts der Dialysator-Blutkammer (34) angeordnet ist.

9. Hämodialysegerät (10) nach einem der vorangegangenen Ansprüche, wobei das Leitungsventil (41,51) als elektrisches Klemmventil ausgebildet ist.

10. Hämodialysegerät (10) nach einem der vorangegangenen Ansprüche, wobei fluidisch zwischen der Förderpumpe (24) und der Dialysatkammer (32) des Dialysators (30) einen Sterifilter (28) angeordnet ist.

11. Hämodialysegerät (10) nach einem der vorangegangenen Ansprüche, wobei die Förderpumpe (24) und die Abfallpumpe (26) miteinander gekoppelt sind und zusammen eine Bilanzierpumpe (27) bilden.

## Claims

1. Hemodialysis device (10) for performing a single-needle hemodialysis treatment, comprising a dialysate circuit (12), a blood circuit (14) and a dialyzer (30) between the dialysate circuit (12) and the blood circuit (14),
wherein the dialyzer (30) has a dialysate chamber (32) and a blood chamber (34),
wherein the dialysate circuit (12) comprises:
a dialysate source (20) which supplies a dialysis liquid,
a feed pump (24) between the dialysate source (20) and the dialyzer (30) for feeding the dialysis liquid form the dialysate source (20) to a dialysate chamber (32) of the dialyzer (30), and
a waste pump (26) downstream of the dialysate chamber (32) of the dialyzer (30), wherein the dialysate chamber (32) and the waste pump (26) are in fluid connection through an outflow line (22),
wherein the blood circuit (14) comprises:
a single vessel connection line (48) which is adjoined by a line branching (47) from which a suction line (40) leads to an inlet of the dialyzer blood chamber (34) and a return line (50) leads to an outlet of the dialyzer blood chamber (34),
a blood pump (44) along the suction line (40), and
a line valve (51) along the return line (50),
wherein a separate oscillation volume (60') branched from the outflow line (22) is arranged in the dialysate circuit (12) and fluidically between the feed pump (24) and the waste pump (26), and
wherein a device control is provided which coordinates the switching of the line valve (51) and the oscillation of the oscillation volume.

2. Hemodialysis device (10) of claim 1, wherein the oscillation volume (60') is defined by a driven oscillation pump (60).

3. Hemodialysis device (10) of claim 1 or 2, wherein the device control comprises a synchronizer (70) controlling the line valve (52) and the oscillation pump (60) with the same duty cycle so that the closing frequency of the line valve (51) and the oscillation frequency of the oscillation pump (60) are the same.

4. Hemodialysis device (10) of claim 3, wherein the oscillation pump (60) is designed as a stroke pump with a constant maximum lifting volume.

5. Hemodialysis device (10) of claim 4, wherein the oscillation pump (60) is designed as a piston pump or as a membrane pump.

6. Hemodialysis device (10) of one of the preceding claims, wherein the oscillation pump (50) operates at a constant displace volume that is 0.5 ml to 100 ml, particularly preferred 3.0 ml - 30 ml.

7. Hemodialysis device (10) of one of the preceding claims, wherein the blood pump (44) is designed as a peristaltic hose pump.

8. Hemodialysis device (10) of one of the preceding claims, wherein a second line valve (41) is arranged on the suction line (40) upstream of the dialyzer blood chamber (34).

9. Hemodialysis device (10) of one of the preceding claims, wherein the line valve (41, 51) is designed as an electric clamping valve.

10. Hemodialysis device (10) of one of the preceding claims, wherein a sterile filter (28) is arranged fluidically between the feed pump (24) and the dialysate chamber (32) of the dialyzer (30).

11. Hemodialysis device (10) of one of the preceding claims, wherein the feed pump (24) and the waste pump (26) are coupled with each other and together form a balancing pump (27).

## Revendications

1. Appareil d'hémodialyse (10) destiné à l'exécution d'un traitement d'hémodialyse du type "single needle", avec un circuit de dialysat (12), un circuit de sang (14) et un dialyseur (30) entre le circuit de dialysat (12) et le circuit de sang (14),
le dialyseur (30) comprenant une chambre de dialysat (32) et une chambre de sang (34),
le circuit de dialysat (12) comprenant:
une source de dialysat (20) fournissant un liquide de dialyse,
une pompe d'alimentation (24) entre la source de dialysat (20) et le dialyseur (30) pour fournir le liquide de dialyse de la source de dialysat (20) vers la chambre de dialysat (32) du dialyseur (30), et
une pompe à déchets (26) en aval de la chambre de dialysat (32) du dialyseur (30), la chambre de dialysat (32) et pompe à déchets (26) étant en liaison fluidique à travers une conduite de sortie (22),
le circuit de sang (14) comprenant:
une seule conduite d'abouchement à un vaisseau sanguin (48) suivie par un branchement (47) de la conduite, à partir duquel une conduite d'aspiration (40) mène à une entrée de la chambre de sang (34) du dialyseur et une conduite de recirculation (50) mène à une sortie de la chambre de sang (34) du dialyseur,
une pompe à sang (44) sur le parcours de la conduite d'aspiration (40), et
une vanne de conduite (51) sur le parcours de la conduite de recirculation (50),
un volume d'oscillation (60') séparé, branché de la conduite de sortie (22), est disposé dans le circuit de dialysat (12) et fluidiquement entre la pompe d'alimentation (24) et la pompe à déchets (26), et
une commande d'appareil est prévue concordant la commutation de la vanne de conduite (51) et l'oscillation dudit volume d'oscillation.

2. Appareil d'hémodialyse (10) selon la revendication 1, dans lequel le volume d'oscillation (60') est formé par une pompe d'oscillation (60) entraînée.

3. Appareil d'hémodialyse (10) selon la revendication 1 ou 2, dans lequel la commande d'appareil comprend un synchroniseur (70) commandant la vanne de conduite (51) et pompe d'oscillation (60) avec la même cadence de sorte que la fréquence de fermeture de la vanne de conduite (51) et la fréquence d'oscillation de la pompe d'oscillation (60) soient égales.

4. Appareil d'hémodialyse (10) selon la revendication 3, dans lequel la pompe d'oscillation (60) est en forme d'une pompe de soulèvement avec un volume de soulèvement maximal constant.

5. Appareil d'hémodialyse (10) selon la revendication 4, dans lequel la pompe d'oscillation (60) est en forme d'une pompe à piston ou d'une pompe à membrane.

6. Appareil d'hémodialyse (10) selon l'une quelconque des revendications précédentes, dans lequel la pompe d'oscillation (60) opère avec un volume de soulèvement constant de 0,5 ml à 100 ml, particulièrement préféré 3,0 ml - 30 ml.

7. Appareil d'hémodialyse (10) selon l'une quelconque des revendications précédentes, dans lequel la pompe à sang (44) est réalisée en forme d'une pompe péristaltique

8. Appareil d'hémodialyse (10) selon l'une quelconque des revendications précédentes, dans lequel une deuxième vanne de conduite (41) est disposée sur la conduite d'aspiration (40) en amont de la chambre de sang (34) dudit dialyseur.

9. Appareil d'hémodialyse (10) selon l'une quelconque des revendications précédentes, dans lequel la vanne de conduite (41, 51) est conçue comme une soupape à pincement électrique.

10. Appareil d'hémodialyse (10) selon l'une quelconque des revendications précédentes, dans lequel un filtre stérile (28) est disposé fluidiquement entre la pompe d'alimentation (24) et la chambre à dialysat (32) du dialyseur (30).

11. Appareil d'hémodialyse (10) selon l'une quelconque des revendications précédentes, dans lequel la pompe d'alimentation (24) et la pompe à déchets (26) sont couplées l'une à l'autre et ensemble forment une pompe bilancière (27).
